Europäisches Patentamt

European Patent Office

Office européen des brevets

(11) Numéro de publication: **0 317 408**
A1

## (12) DEMANDE DE BREVET EUROPEEN

(21) Numéro de dépôt: **88402841.6**

(51) Int. Cl.⁴: **A 61 F 2/08**

(22) Date de dépôt: **10.11.88**

(30) Priorité: **13.11.87 FR 8715900**

(43) Date de publication de la demande:
**24.05.89 Bulletin 89/21**

(84) Etats contractants désignés:
**AT BE CH DE ES FR GB GR IT LI LU NL SE**

(71) Demandeur: **IMPLANTS MEDICAL SYSTEME, S.a.r.l.**
**270 Avenue de Lardenne**
**F-31100 Toulouse (FR)**

(72) Inventeur: **D'Arzac, Pierre, Dr.**
**Chemin le Plot§Le Pignadou§Villemoustousou**
**F-11600 Conques Sur Orbiel (FR)**

(74) Mandataire: **Ravina, Bernard**
**Cabinet Bernard RAVINA 24, boulevard Riquet**
**F-31000 Toulouse (FR)**

(54) Prothèse ligamentaire ou tendineuse et procédé de fabrication de la dite prothèse.

(57) La présente invention concerne une prothèse ligamentaire ou tendineuse et également le procédé de fabrication de celle-ci.

La prothèse selon l'invention est formée à partir de faisceaux (1) de fibres de matière biocompatible, est dotée à une de ses extrémités d'un moyen de blocage (2) et à son autre extrémité d'au moins un moyen de fixation (3) dans l'os et se caractérise en ce qu'elle comporte au moins une tresse constituée d'une pluralité de faisceaux et dont les extrémités sont solidaires pour former une boucle, au moins une première gaine (5) entourant au moins la moitié de la longueur de la ou des tresses, au moins une seconde gaine (6) tressée en continuité de la ou des premières gaines (5) et enveloppant la ou les tresses et la ou les dites premières gaines de façon à former au moins un oeillet (7) destiné à recevoir des moyens de fixation dans l'os et en ce que les extrémités de la ou des tresses, les extrémités de la ou des première et seconde gaines (5) et (6) sont fixées ensemble par rapport au moyen de blocage (2).

Fig. 1

EP 0 317 408 A1

**Description**

## PROTHESE LIGAMENTAIRE OU TENDINEUSE ET PROCEDE DE FABRICATION DE LA DITE PROTHESE.

La présente invention a pour objet une prothèse ligamentaire ou tendineuse et concerne également le procédé de fabrication de la dite prothèse.

La prothèse selon l'invention est destinée à être implantée sur un patient par une méthode chirurgicale connue en vue du remplacement ou du renforcement d'un ligament ou d'un tendon déficient.

De façon connue les prothèse implantées à cet effet, sont constituées de plusieurs faisceaux de fibres de matière biocompatible identiques ou différents qui sont tressés.

La matière biocompatible utilisée dans les prothèse de l'art antérieur est soit du polyester, soit une matière synthétique acrylique connue sous le nom commercial de Dacron, soit encore du carbone pur.

Ces prothèse de l'art antérieur présentent plusieurs inconvénients quant à leur constitution, à leur mise en place et aux résultats qu'elles procurent après leur implantation.

En effet, certaines risquent d'être détériorées par effilochage et rupture de fibres carbone lors de leur mise en place, d'autres ne possédent pas ou peu de point d'ancrage efficace dans le temps, ou encore présentent une dimension transversale importante faisant appel à des opérations préparatoires du lieu d'implantation longues et délicates.

En effet, dans le cadre d'un remplacement ou renforcement d'un certain type de ligament tel que les ligaments croisés du genou, la méthode d'implantation de ces prothèses comporte une opération de perçage de la tete du tibia et/ou du condyle fémoral.

On comprendra aisément que plus la dimension transversale de la prothèse est grande plus l'enlèvement de matière est important, ceci nécessite donc une instrumentation plus volumineuse et entraîne une plus grande fragilisation des tissus osseux, ainsi qu'une rééducation plus longue par ailleurs.

D'autre part, il s'avère que les fibres des prothèses se cassent par frottement contre les os. Cette destruction des fibres entraine la diminution de la résistance à la traction de la prothèse et provoque également pour des prothèses sur lesquelles les faisceaux de fibres de carbone sont à nu la détérioration de ces fibres qui ont tendance à se regrouper pour former des nodules pouvant entrainer des réactions pathologiques.

Par ailleurs, les prothèses connues jusqu'alors utilisent certes des matériaux biocompatibles mais ne sont pas dotées de moyens de blocage dans l'os permettant une réhabilitation de celui-ci totale et rapide.

Ceci entraine l'existence d'une zone d'insertion de résistance plus faible et nécessite une immobilisation de l'articulation du patient longue.

La présente invention vise à obvier à l'ensemble de ces inconvénients en proposant une prothèse offrant une rigidité relative pour une bonne tenue sur toute sa longueur, présentant une dimension transversale réduite sans que sa résistance à la traction en soit altérée et facilitant ainsi la mise en place, permettant une réhabilitation de l'os rapide dans la zone de blocage et présentant une protection des fibres la constituant pour éviter leur destruction lors de l'implantation.

A cet effet, la prothèse selon l'invention est formée à partir de faisceaux de fibres de matière biocompatible, est dotée sur une de ses extrémités d'un moyen de blocage et sur son autre extrémité d'au moins un moyen de fixation dans l'os et se caractérise en ce qu'elle comporte au moins une tresse constituée d'une pluralité de faisceaux et dont les extrémités sont solidarisées pour former une boucle, au moins une première gaine tressée entourant au moins la moitié de la longueur de la ou des tresses, au moins une seconde gaine tressée en continuité de la ou des premières gaines et enveloppant la ou les tresses et la ou les premières gaines de façon à former au moins un oeillet destiné à recevoir s moyens de fixation dans l'os et en ce que les extrémités de la ou des gaines, la ou les extrémités de la ou des premières et secondes gaines sont fixées ensemble par rapport au moyen de blocage.

Selon une autre caractéristique de la prothèse selon l'invention le moyen de blocage est constitué par du carbonate de calcium d'origine madréporaire à structure ajourée ou corail.

D'autres avantages et caractéristiques apparaitront à la lecture de la description de l'invention illustrée aux dessins annexés donnés à titre d'exemple non limitatif et en lesquels :

    - la fig. 1 est une vue en perspective de la prothèse selon l'invention.

    - la fig. 2 est une vue schématique en coupe transversale de la dite prothèse.

    - la fig. 3 est une vue en coupe du moyen de blocage de la prothèse selon l'invention.

    - la fig. 4 est une vue schématique en coupe longitudinale de la dite prothèse.

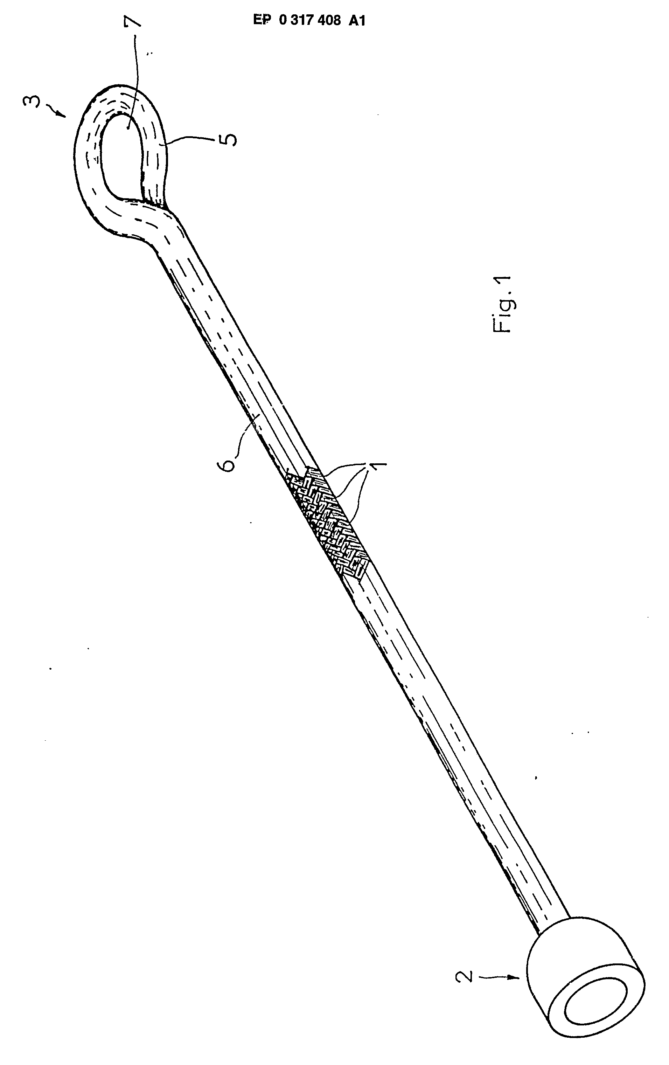

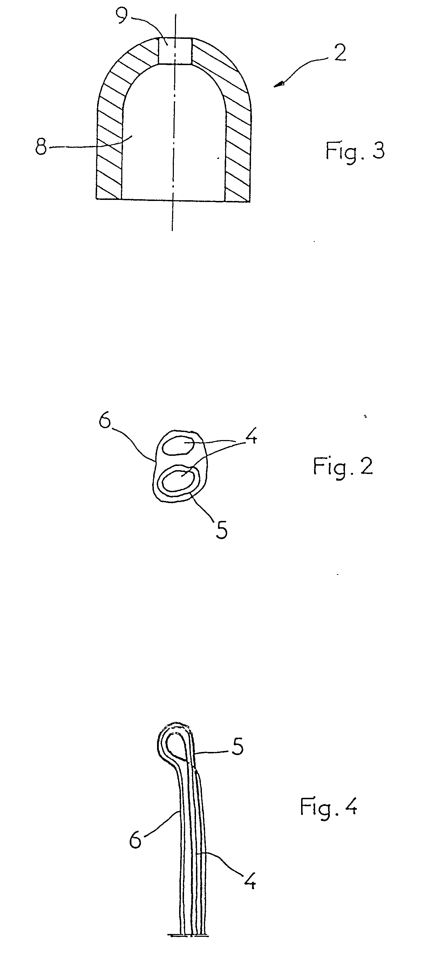

La prothèse ligamentaire ou tendineuse selon la présente invention telle que représentée aux figures 1 et 2 est formée à partir de faisceaux 1 de fibres de matière biocompatible.

La dite matière biocompatible est constituée de façon connue soit par du polyester, soit par une matière synthétique acrylique connue sous le nom déposé de "Dacron", soit encore par du carbone pur.

La prothèse est formée d'une pluralité de faisceaux de même matière ou de matière distincte.

La dite prothèse est pourvue sur une de ses extrémités d'un moyen de blocage 2 et sur son autre extrémité d'au moins un moyen de fixation 3 dans l'os.

La prothèse selon l'invention comporte au moins une tresse 4 constituée d'une pluralité de faisceaux 1 et dont les extrémités sont solidarisées de façon à former une boucle, au moins une première gaine 5 tressée entourant au moins la moitié de la longueur de la ou des tresses 4, au moins une seconde gaine 6 tressée en continuité de la ou les premières gaines 5 et enveloppant la ou les tresses 4 et la ou les dites premières gaines de façon à former au moins un oeillet 7 recevant des moyens de fixation 3 dans l'os.

Les extrémités de la ou des tresses 4, la ou les extrémités de la ou des premières gaines 5 et de la ou des secondes gaines 6 sont fixées ensemble par rapport au moyen de blocage 2.

Suivant une forme de réalisation préférentielle, la prothèse selon l'invention comporte une tresse 4 formant une boucle, une première gaine 5 entourant la tresse 4 sur au moins la moitié de sa longueur à partir d'une extrémité de celle-ci et une seconde gaine 6 recouvrant la tresse 4 et la première gaine 5 pour réaliser un oeillet 7.

Les extrémités de la tresse 4, l'extrémité de la première gaine 5 et l'extrémité de la seconde gaine 6 sont fixées ensemble par rapport au moyen de blocage 2.

La tresse 4 est préférentiellement tubulaire et est obtenue par une opération de tressage classique sur une machine de type connu tel que tresseuse verticale ou autres.

La tresse tubulaire 4 est réalisée avec un pas de tressage long de préférence inférieur ou égal à 40 mm, afin que la dite tresse présente un allongement maximum faible de l'ordre de 2% à 4% sous l'effet d'une traction.

Suivant la résistance à la traction que l'on désire obtenir sur la prothèse selon l'invention le nombre de faisceaux 1 et le nombre de fibres par faisceaux 1 varient.

Les dits faisceaux de la tresse 4 sont soit tous en polyester ou en Dacron soit pour certains en polyester ou en Dacron et pour d'autres en carbone pur.

Selon une forme préférentielle de réalisation la tresse est formée de 12 faisceaux en polyester contenant chacun environ 192 fibres et de 4 faisceaux en carbone pur contenant environ 3000 fibres chacun.

Suivant une autre forme de réalisation la tresse 4 peut être formée de 16 faisceaux de polyester ou de Dacron.

Il va de soi que ces nombres ne sont nullement limitatifs.

Cependant il est à noter que la tresse 4 ainsi constituée présente une section transversale réduite.

La tresse 4 comme dit précédemment forme une boucle par assemblage de ses extrémités et est recouverte sur au moins la moitié de sa longueur à partir d'une de ses extrémités d'une première gaine

.

Cette première gaine 5 est réalisée par un tressage classique de préférence identique à celui de la tresse 4 sur une machine de type connue telle que tresseuse.

Le tressage de cette première gaine 5 est réalisée avec un pas d'environ 25 mm afin de contenir la tresse 4 pour protéger les fibres des faisceaux qui la compose et afin de conférer une certaine rigidité à la dite tresse. Cette première gaine 5 présente un allongement maximum sous traction plus important que la tresse 4.

Selon une forme préférentielle, la première gaine 5 est formée avec des faisceaux 1 de même matière et plus particulièrement seulement en polyester ou en Dacron.

Selon la forme de réalisation de la tresse 4 formée avec 12 faisceaux en polyester ou en Dacron et 4 faisceaux en carbone pur ou bien 16 faisceaux en polyester ou en Dacron, la première gaine 5 comporte 16 faisceaux en polyester ou en Dacron.

Préférentiellement, les faisceaux 1 de matière donnée constituant la première gaine 5 contiennent un nombre de fibres identique à celui des faisceaux de la tresse 4 de la même matière.

Il va de soi que le nombre de faisceaux 1 composant la première gaine 5 n'est pas limitatif ainsi que la matière. Cependant, il est préféralbe que la dite première gaine ne contienne pas de faisceaux 1 de fibres de carbone pur afin d'éviter le contact de ces dernières avec les os lors de l'implantation.

La première gaine 5 entoure la tresse 4 au plus proche de celle-ci afin de ne pas augmenter de façon inconsidérée la section transversale de l'ensemble tresse 4 première gaine 5.

Cette première gaine 5 enveloppe la tresse 4 à partir d'une des extrémités de celle-ci et sur au moins la moitié de la longueur de la dite tresse.
Préférentiellement, la première gaine 5 enveloppe la tresse 4 au delà de la moitié de la longueur de celle-ci.

La prothèse selon l'invention comporte également une seconde gaine 6 qui entoure la tresse 4 et la première gaine 5. Cette seconde gaine 6 est réalisée en continuite de la première gaine 5 en reprenant les faisceaux 1 de celle-ci et en formant une tresse tubulaire autour de la portion de la tresse 4 qui n'est pas recouverte par la première gaine 5 et de la dite première gaine entourant une portion de la tresse 4 tel que représenté en fig. 2.

La tresse 4 recouverte en partie par la première gaine 5 forme une boucle largement ouverte et qui est resserrée lors de la réalisation de la seconde gaine 6 pour former l'oeillet 7.

La longueur de la première gaine 5 prise au delà de la moitié de la longueur de la tresse 4 définit la longueur périphérique de l'oeillet.

Le tressage de la seconde gaine 6 est de type classique et de préférence identique à celui de la première gaine 5 et est réalisé sur une machine connue telle que tresseuse verticale.
La dite seconde gaine 6 est réalisée avec un pas de tressagee inférieur à celui de la première gaine 5 et de l'ordre de 13 mm afin de conférer à la prothèse une bonne tenue sur elle-même.
Le pas de tressage de la seconde gaine 6 ne peut être inférieur à une certaine limite afin de maintenir sur la dite seconde gaine une perméabilité au tissu osseux pour la réhabilitation de celui-ci.

Le diamètre de la seconde gaine 6 est suffisant pour contenir la tresse 4 et la première gaine 5 et présente une valeur réduite.

Le pas de tressage de la tresse 4 est supérieur aux pas de tressage des première et seconde gaines 5 et 6 et présente donc un allongement inférieur aux dites première et seconde gaines sous traction.

Cette tresse 4 est donc l'élément de la prothèse selon l'invention qui supporte tous les efforts de traction. La première gaine 5 permet de protéger une portion de tresse 4 au niveau de l'oeil let et la seconde tresse 6 protège la portion de la tresse 4 non entourée par la première gaine 5.

Les dites première et seconde gaines permettent à la prothèse de présenter une certaine rigidité lui donnant une bonne tenue sur elle-même.

Les première et seconde gaines présentent également un pas de tressage leur conférant une certaine perméabilité facilitant la réhabilitation du tissu osseux. Cette réhabilitation est d'autant plus facilitée au niveau de la tresse 4 par la présence dans celle-ci des faisceaux de fibres de carbone pur.

Comme énoncé précédemment, les extrémités de la tresse 4, l'extrémité de la première gaine 5 et l'extrémité de la seconde gaine 6 sont fixées ensemble par rapport au moyen de blocage 2.

Le dit moyen de blocage de la prothèse selon l'invention tel que représentée en figure 3 présente un profil dont les sections transversales diminuent suivant une direction.

Selon une forme préférentielle de réalisation le moyen de blocage 2 présente une portion cylindrique et une portion sphérique offrant la diminution de section transversale.

Mais, il va de soi que ce moyen de blocage ne se limite pas à cette forme de réalisation et peut être en tronc de cône, ou bien en pyramide tronquée.

La diminution de la section permet une pénétration aisée du moyen de blocage 2 dans le dégagement ménagé dans l'os pour la mise en place de la prothèse sur un patient.
Préférentiellement, le profil du dégagement ménagé dans l'os est sensiblement identique au profil du moyen de blocage 2.

Ce moyen de blocage présente un évidement 8 de profil concentrique au profil extérieur et un orifice 9 ménagé dans son axe longitudinal et au niveau de la section de plus faible dimension.

Selon la forme de réalisation de la figure 3, l'orifice 9 est ménagé dans la portion sphérique et coaxialement à l'axe de révolution du moyen de blocage.

La tresse 4 enveloppée en partie par la première gaine 5 et par la seconde gaine 6 passe par l'orifice 9 et débouche en avant de la portion sphérique et les extrémités de la dite tresse, l'extremité de la première gaine 5 et l'extrémité de la seconde gaine 6 sont bloquées dans l'évidement 8 du moyen de blocage 2.

A cet effet, les extrémités de la tresse 4 peuvent être nouées ensemble pour former un obstacle vis à vis de l'orifice 9, les première et seconde gaines 5 et 6 étant disposées autour du noeud réalisé dans l'évidement 8.

Avantageusement, le dit évidement est rempli d'une matière adhérente afin de bloquer définitivement la tresse 4 les première et seconde gaines 5 et 6 par rapport au moyen de blocage 2.
Cette matière adhérente est préférentiellement constituée par un cément osseux à prise rapide de type connu.

Les extrémités de la tresse 4 étant bloquées ensemble les deux brins de la boucle formée par la dite tresse ne peuvent avoir de mouvement relatif l'un par rapport à l'autre lorsqu'ils sont enveloppés par les première et seconde gaines 5 et 6.

L'extrémité de la première gaine 5 et l'extrémité de la seconde gaine 6, réalisée en continuité de la dite première gaine et enveloppant cette dernière, étant bloquées ensemble également, ne peuvent avoir de mouvement relatif l'une par rapport à l'autre.

La tresse 4 présentant un pas de tressage plus long que les première et seconde gaines 5 et 6 et donc un allongement moindre que ces dernières sous une traction, il ne peut y avoir de mouvement entre la dite tresse et les dites gaines.

Le moyen de blocage 2 de la prothèse selon l'invention est constitué par du carbonate de calcium par exemple d'origine madréporaire à structure ajourée ou corail.

La matière constituant le moyen de blocage 2 permet une réhabilitation des cellules osseuses très rapide formant après un certain temps un cal cicatriciel osseux particulièrement résistant.

De plus, cette réahabilitation est totale et rapide au niveau du moyen de blocage grâce également à la présence du cément osseux dans lequel sont noyées la tresse 4 et les première et seconde gaines 5 et 6.

La mise en place de la prothèse selon l'invention se fait suivant des procédés chirurgicaux connus consistant à faire passer l'oeillet 7 à travers le trou préalablement ménagé dans l'os, à placer le moyen de blocage 2 dans le dégagement réalisé à l'entrée du trou dans la zone corticale de l'os, à tendre la prothèse et à fixer cette dernière en plaçant dans l'oeillet 7 des éléments de fixation adaptés.

Ces éléments de fixation sont constitués par une rondelle dotée à sa périphérie extérieure d'une rainure et destinée à se loger dans l'oeillet 7 et à se bloquer dans celui-ci par sa rainure et par une vis traversant la rondelle et se vissant dans les zones corticales de part et d'autre de la zone médullaire de l'os.

Cette mise en place de la prothèse est d'autant plus facilitée par sa bonne tenue sur elle-même et évite le retrait d'une trop grande quantité de matière osseuse grâce à sa faible section transversale.

La prothèse selon l'invention est réalisée suivant un procédé de fabrication qui consiste à réaliser une tresse 4 de longueur égale au double de la longueur de la prothèse que l'on désire, à réaliser autour de cette tresse 4 une première gaine 5 sur une longueur supérieure à la moitié de la longueur de la tresse 4, à ramener l'extrémité de la tresse 4 gainée contre l'extrémité de la tresse 4 non gainée, à réaliser dans la continuité de la première gaine 5 la seconde gaine 6 autour de la tresse 4 non gainée et de la première gaine 5 afin de former un oeillet 7, à couper la gaine 6 au niveau des extrémités de la tresse 4, à enfiler le moyen de blocage 2 sur la prothèse ainsi obtenue et à bloquer les extrémités de la tresse 4, l'extrémité de la première gaine 5 et de la seconde gaine 6 par rapport au dit moyen de blocage.

Selon un mode préférentiel de réalisation du procédé la tresse 4 est réalisée sur une très grande longueur sur une première machine à tresser puis est coupée à la longueur désirée avec la seconde gaine 6.

Les premières et seconde gaines 5 et 6 sont réalisées sur une autre machine à tresser dans laquelle est introduite l'extrémité de la tresse 4 de grande longueur.

La longueur de la première gaine 5 réalisée au delà de la moitié de la longueur de la tresse 4 définit la longueur de la circonférence de l'oeillet 7.

Lorsque la tresse est réalisée sur une très grande longueur, la portion de celle-ci entourée par la première gaine 5 qui est repliée présente une longueur égale à celle de la prothèse que l'on désire obtenir.

Donc le procédé selon l'invention utilise préférentiellement une première machine à tresser pour réaliser la tresse 4 et une deuxième machine à tresser pour réaliser les première et seconde gaines 5 et 6.
Mais il va de soi, que la tresse 4 et les dites première et seconde gaines 5 et 6 peuvent être réalisées sur une même machine à tresser.

D'autre part, entre la fin de la réalisation de la première gaine 5 et le début de la réalisation de la seconde gaine 6, le pas de tressage doit être modifié.

Le blocage des extrémités de la tresse 4 et de l'extrémité des première et seconde gaines 5 et 6 est réalisé en formant sur celles-ci des noeuds et en remplissant le dit moyen de blocage d'une matière adhérente.

Le procédé selon l'invention prévoit également après réalisation de la prothèse de stériliser celle-ci par toute méthode appropriée par exemple physico-chimique ou autres.

La prothèse selon l'invention permet une réhabilitation rapide et totale sur un patient, est de conception aisée et est réalisée à partir d'un procédé de mise en oeuvre simple et peu coûteux.

Il va de soi que la présente invention, peut recevoir tout aménagement et toute variante dans le domaine des équivalents techniques sans pour autant sortir du cadre du présent brevet.

## Revendications

R1) Prothèse ligamentaire ou tendineuse formée à partir de faisceaux (1) de fibres de matière biocompatible tel que polyester, "Dacron", ou carbone pur et dotée sur une de ses extrémités d'un moyen de blocage (2) et sur son autre extrémité d'au moins un moyen de fixation (3) dans l'os, caractérisée en ce qu'elle comporte au moins une tresse (4) constituée d'une pluralité de faisceaux (1) et dont les extrémités sont solidarisées de façon à former une boucle, au moins une première gaine (5) tressée entourant au moins la moitié de la longueur de la ou des tresses (4), au moins une seconde gaine (6) tressée en continuité de la ou des premières gaines (5) et enveloppant la ou les tresses (4) et la ou les premières gaines (5) de façon à former au moins un oeillet (7) recevant des moyens de fixation (3) dans l'os et en ce que les extrémités de la ou des tresses (4), la ou les extrémités de la ou des premières gaines (5) et de la ou des secondes gaines (6) sont fixées ensemble par rapport au moyen de blocage (2).

R2) Prothèse selon la revendication 1 caractérisée en ce que la tresse (4) présente un pas de tressage supérieur au pas de tressage de la première gaine (5) et la dite première gaine présente un pas de tressage supérieur au pas de tressage de la seconde gaine (6).

R3) Prothèse selon les revendications 1 et 2 caractérisée en ce que la tresse (4), la première gaine (5) et la seconde gaine (6) sont tubulaires.

R4) Prothèse selon la revendication 1 caractérisée en ce que la première gaine (5) entoure la tresse (4) au delà de la moitié de la longueur de celle-ci.

R5) Prothèse selon la revendication 1 caractérisée en ce que le moyen de blocage (2) est constitué par du carbonate de calcium d'origine madréporaire à structure ajourée.

R6) Prothèse selon les revendications 1 et 5 dont le moyen de blocage (2) est doté d'un évidement (8) et d'un orifice (9), caractérisée en ce que les extrémités de la tresse (4), l'extrémité de la première gaine (5) et de la seconde gaine (6) sont logées dans l'évidement (8) et bloquées par rapport à l'orifice (9) du moyen de blocage et en ce que l'évidement (8) est empli d'une matière adhérente noyant les extrémités de la tresse (4) et des première et seconde gaines (5) et (6).

R7) Prothèse selon les revendications 1 et 6 caractérisée en ce que la matière adhérente emplissant l'évidement (8) du moyen de blocage (2) est du cément osseux.

R8) Procédé de fabrication de la prothèse selon les revendications 1 à 7 caractérisé en ce qu'il consiste à réaliser une tresse (4) de longueur égale au double de la longueur de la prothèse que l'on désire, à réaliser autour de cette tresse (4) une première gaine (5) sur une longueur supérieure à la moitié de la longueur de la tresse (4), à ramener l'extrémité de la tresse (4) gainée contre l'extrémité de la tresse (4) non gainée, à réaliser dans la continuité de la première gaine (5) la seconde gaine (6) autour de la tresse (4) non gainée et de la première gaine (5) afin de former un oeillet (7), à couper la gaine (6) au niveau des extrémités de la tresse (4), à enfiler le moyen de blocage (2) sur la prothèse ainsi obtenue, et à bloquer les extrémités de la tresse (4), l'extrémité de la première gaine (5) et de la seconde gaine (6) par rapport au dit moyen de blocage.

R9) Procédé selon la revendication 8 caractérisé en ce que la tresse (4) est réalisée sur une très grande longueur puis est coupée à la longueur voulue avec la seconde gaine (6).

Fig. 1

EP 0 317 408 A1

9

2

8

Fig. 3

6

4

Fig. 2

5

5

6

Fig. 4

4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 238 263 (RICHARDS MEDICAL CO.)<br>* Page 6, ligne 17 - page 10, ligne 28; figures * <br>--- | 1,3,8 | A 61 F   2/08 |
| A | SURGERY, vol. 1, no. 34, juillet 1986, pages 808-811, Medical Education (International) Ltd, Oxford, GB; A.A. AMIS: "Artificial ligaments"<br>* Page 808; figure *<br>--- | 1 | |
| A | FR-A-1 046 555 (MERON)<br>--- | | |
| A | EP-A-0 201 905 (HAUER)<br>------ | | |

DOMAINES TECHNIQUES RECHERCHES (Int. Cl.4)

A 61 F
A 61 B

Le présent rapport a été établi pour toutes les revendications

| Lieu de la recherche | Date d'achèvement de la recherche | Examinateur |
|---|---|---|
| LA HAYE | 07-02-1989 | STEENBAKKER J. |